(19) Europäisches Patentamt
European Patent Office
Office européen des brevets

(11) EP 1 958 584 A1

(12) **EUROPEAN PATENT APPLICATION**

(43) Date of publication:
 **20.08.2008 Bulletin 2008/34**

(51) Int Cl.:
 **A61B 18/22** (2006.01)  **A61B 17/00** (2006.01)

(21) Application number: **07003129.9**

(22) Date of filing: **14.02.2007**

(84) Designated Contracting States:
 **AT BE BG CH CY CZ DE DK EE ES FI FR GB GR HU IE IS IT LI LT LU LV MC NL PL PT RO SE SI SK TR**
 Designated Extension States:
 **AL BA HR MK RS**

(71) Applicant: **Sade, Sharon**
 **Kefar Yona (IL)**

(72) Inventor: **Sade, Sharon**
 **Kefar Yona (IL)**

(74) Representative: **Arth, Hans-Lothar et al**
 **ABK Patent Attorneys**
 **Jasminweg 9**
 **14052 Berlin (DE)**

(54) **Temperature controlled multi-wavelength laser welding and heating system**

(57) A temperature controlled welding, soldering and heating system having a plurality of light sources of different wavelengths for simultaneously irradiating a tissue sample and an IR radiometer for providing tissue sample temperature reading in real time, the temperature readings used for controlling the light sources. In a preferred embodiment, the sample is a tissue sample and the light sources are lasers.

**FIGURE 1A**

**100**

**Description**

FIELD OF THE INVENTION

The present invention relates generally to light source or laser welding, soldering and heating of tissue samples and more particularly to temperature controlled laser welding, soldering and heating systems applied to tissue welding.

BACKGROUND OF THE INVENTION

Tissue sample heating and welding by light (e.g. laser) irradiation is known. In particular, tissue heating by light irradiation is used in many medical and surgical applications such as laser welding of tissues, laser assisted cartilage reshaping, and in laser hyperthermia procedures. The light energy is absorbed by the tissue and causes local heat. These clinical applications are highly dependent on defining and maintaining the optimal conditions of the light source. Irreversible thermal damage to the tissue by direct laser heating and heat transfer is of a particular concern. Therefore, the general objective of laser-tissue interaction is to accurately heat a desired volume with minimal effects in the surroundings. Since the thermal effect is fundamental to the mechanism behind laser tissue interaction, temperature control is essential to achieve a successful clinical procedure.

Laser welding of tissues is described in a number of references, see e.g. Karen M.McNally-Heintzelman and Ashley J.Welch, "Laser Tissue Welding", chapter 39 in Biomedical Photonics Handbook, Tuan Vo-Dinh, 2003 and L.S.Bass and M.R. Treat, "Laser Tissue Welding: A Comprehensive Review of Current and Future Clinical Applications", Lasers in Surgery and Medicine vol. 17, pp.315-349, 1995. This is an experimental technique for tissue closure that offers many advantages over conventional closure techniques, such as reduced suture and needle trauma, reduced foreign body reaction, better cosmetic appearance, reduce bleeding, immediate water tight closure and shorting operating times.

Many in-vitro and in-vivo experimental studies were carried out to weld various types of tissues by using different lasers. An exemplary method and system of controlled laser welding of tissue is disclosed in US Patent No. 5,409,481. A tissue sample is heated controllably while irradiated by one laser source having one wavelength only. A method and system employing a fiber laser device for medical and cosmetic procedures is disclosed in US Patent Application No. 2003/0055413. The wavelength of a tunable laser source is changed and tuned to a desired wavelength and then the tissue is irradiated in this specific wavelength. A method and system employing an active laser gain medium for medical procedures is also disclosed in US Patent No. 6,162,213. An active gain medium comprising metal vapor is excited to produce a plurality of wavelengths used in a medical procedure.

Different types of light activated surgical biological adhesives and stents were developed and used to assist the weld procedure and strength, see e.g. US patents 6,607,522, 5,552,452, and 4,633,870. It is assumed that the welding is achieved due to thermal restructuring within the tissue collagen, where new bonds and interaction with adjacent proteins are formed and stabilized upon cooling. A narrow margin exists between a successful and unsuccessful weld, since the process is highly dependent on the temperature.

The temperature of a tissue sample can be determined by measuring the thermal infrared radiation emitted from it, because warm objects emit IR thermal radiation whose intensity is dependent on their surface temperature. The total intensity I, of the radiation emitted from a surface is given by the equation $I = \varepsilon \sigma T^4$, where $\varepsilon$ is the emissivity which depends on the sample type and surface quality, $\sigma$ is the Stefan-Boltzman constant and T is the temperature.

The wavelength of maximal emission $\lambda_m$ is related to the heated body temperature T by Wien's displacement Law: $\lambda_m T = 2898$ $\mu$m K. For biological tissue, T is roughly 300K. It follows that the spectral range of interest for such low temperature radiometry is in the middle infrared (mid-IR) in the spectral range 3-30 $\mu$m. The infrared emission emitted from a source can be measured by an infrared radiometer and calibrated for determining the temperature of the source. IR radiometers that measure the radiation emitted from a distant source [see e.g. S. Sade, O. Eyal, V. Scharf and A. Katzir, "Fiberoptic Infrared Radiometer for Accurate Temperature Measurements," Applied Optics, Vol. 41, no. 10, pp. 1908-1914, 2002] usually consist of three parts: (1) optics to collect the radiation and to focus it on a detector, (2) an infrared detector to convert the radiation to an electric signal, (3) an electronic system for processing the signal.

Only a few optical fibers are transparent in the IR range. Optical fibers made of silver halides are among the best candidates for that purpose. They are highly transparent in the mid-infrared, in the spectral range 3-30 $\mu$m, with losses of about 0.2dB/m at 10.6 $\mu$m. IR radiometers which use optical fibers to collect and deliver the radiation to the detector are called IR fiber optic radiometers [see e.g. A Zur and A. Katzir, "Use of infrared fibers for low temperature radiometric measurements", Applied Physics Letters, vol. 48, p.449, 1986]. Such a radiometer has an advantage relative to other instruments used to measure temperature, such as thermocouples; the measurement can be done in an electromagnetic environment, in situation in which there is no clear line of sight to the measured sample, and in endoscopic procedures.

The IR detectors used in the radiometers may be thermal detectors, such as pyroelectric, thermoresistive and thermopile devices, many of whom operate at room temperature, or photonic (i.e. quantum, photoconductive or photovoltaic) detectors such as MCT (HgCdTe) or InSb, which are cooled by liquid nitrogen or thermoelectrically.

Some prior art tissue heating and/or welding methods are disadvantageous in that the tissue sample is irradiated by only one laser source having only one wavelength. Other prior art methods do not control and monitor the temperature of the tissue sample or do not irradiate the tissue sample simultaneously with several light sources, each having a different wavelength. Consequently, the upper layers of the tissue sample are being heated and a temperature gradient is formed inside the tissue, preventing tissue welding inside the tissue and/or causing thermal irreversible damage to the tissue.

There is therefore a widely recognized need for, and it would be highly advantageous to have a welding, soldering and heating system and method in which a sample such as a tissue sample is simultaneously irradiated by a plurality of light sources, preferably lasers, each having different wavelength, while measuring the temperature of the tissue sample, to controllably heat the tissue sample and which temperature is monitored in real time using an IR radiometry subsystem that provides respective IR radiation inputs to control the lasers.

## SUMMARY OF THE INVENTION

We propose and demonstrate, for the first time, a temperature controlled welding, soldering and heating system which uses simultaneously a plurality of light sources, preferably lasers, each having a different wavelength, imposed to irradiate a tissue sample. In a preferred embodiment the sample is a tissue sample. However, in a general sense, the present invention is equally applicable to other types of solid samples such as plastic, metal and semi-conductor samples. The advantage of such a system is as follows: a better control of the heated tissue sample can be achieved by using several lasers simultaneously and by providing a closed loop temperature control. Such a temperature controlled multi-laser system is essential for a better heating and welding of biological tissues. For biological tissues the absorption length, and therefore the heated region, is wavelength-dependent and changes according to the tissue type and structure. Therefore by using simultaneously more than one laser wavelength, one can achieve uniformity of the heated region and thus obtain uniform temperature profile inside the tissue. This will enable welding of deep layers of tissues such as deep cuts, skin implants and welding of blood vessels. This can not be done using only one laser having one wavelength or a tunable laser, because of temperature gradients which are formed in the tissue. In order to achieve the temperature uniformity, parameters such as wavelengths, powers, temporal and spatial modulation of the different lasers need to be chosen to fit the tissue type and medical procedure. The light sources should be closely monitored and controlled. This can be achieved by measuring the temperature of the tissue sample, preferably by IR radiometry, which provides inputs to the control system. Moreover, by implementing multi-band IR radiometry, the temperature measurement is immune to emissivity changes of the tissue sample during the irradiation (e.g., the loss of water in the tissue). By irradiating simultaneously the tissue sample by plurality of lasers undesired effects, such as peripheral thermal damage, can be reduced or omitted. Moreover, the multi wavelength system can be tailored to fit any type of biological glue, light-sensitive dyed tissue or cooling liquids.

A system of the present invention includes a plurality of optical light sources each having a different wavelength, which irradiate and heat simultaneously a tissue sample. In one embodiment of the present invention, the system includes an IR radiometer for monitoring the IR radiation emitted from the tissue sample during the heating and for providing respective IR radiation inputs for closed loop control of the plurality of light sources. In some embodiments, the light sources are lasers. In some embodiments, the system may be used for tissue welding. In other embodiments, the system may be used for tissue soldering. In some embodiments, the system includes a single optical port to convey the light source radiation to and the thermal radiation from the tissue sample. In other embodiments, the system includes two ports, one for conveying the light source radiation to the tissue sample and the other for conveying the thermal radiation from the tissue sample. The heated tissue sample emits thermal IR radiation, whose intensity is determined by the temperature of the tissue sample. In some embodiments, the emitted IR radiation can be measured by the IR radiometer in a single spectral band. In other embodiments, the emitted IR radiation can be measured in a plurality of spectral bands. A computer program or similar analyzing means uses the signal to determine the temperature of the tissue sample and to control each of the light sources so that a desirable surface temperature and temperature depth profile is obtained.

According to the present invention there is provided a system for non-contact welding, soldering and heating of a tissue sample including a plurality of light sources each having a different wavelength, the sources operative to simultaneously irradiate the tissue sample thereby causing heating and an IR radiometry subsystem for monitoring the IR radiation emitted from the tissue sample during the heating and for providing respective IR radiation inputs for control of each of the light sources.

According to one feature in the system of the present invention, the light sources include lasers.

According to yet another feature of the present invention, the system further comprising a control unit coupled to both the IR radiometry subsystem and the laser sources and operative to provide the control of each laser source based on the radiation inputs.

According to yet another feature of the present invention, the irradiation by the light sources and the thermal radiation from the tissue sample are conducted through a single port.

According to yet another feature of the present invention, the irradiation by the light sources and the thermal radiation from the tissue sample are conducted through separate ports.

According to yet another feature of the present invention, the single port includes an optical fiber.

According to yet another feature of the present invention, the separate ports include separate optical fibers.

According to yet another feature of the present invention, the lasers are selected from the group consisting of a pulsed laser, a continuous wave gas laser, a solid-state laser, a semiconductor laser and a combination thereof.

According to yet another feature of the present invention, the IR radiometry subsystem includes an IR detector selected from the group consisting of a thermal detector and a photonic detector.

According to yet another feature of the present invention, the IR radiometry subsystem further includes an IR-transparent optical fiber for coupling the IR detector to the tissue sample along an optical path.

According to the present invention there is provided a non-contact welding, soldering and heating system including a plurality of light sources for simultaneously and controllably irradiating and heating a tissue sample, an infrared detector for monitoring IR radiation emitted from the tissue sample during the heating in at least one spectral band and for providing respective IR radiation inputs and a controller for controlling each of the light sources based on the IR radiation inputs, thereby facilitating the controllable heating of the tissue sample.

According to the present invention there is provided a method for controllably welding, soldering and heating of a tissue sample including the steps of: irradiating and heating the tissue sample simultaneously using a plurality of light sources, measuring the temperature of the tissue sample using an IR radiometer in at least one spectral band to provided real-time temperature inputs and controlling each of the light sources based on real-time temperature inputs provided by the IR radiometer, thereby providing a controlled tissue sample temperature.

According to a feature in the method of the present invention, the step of irradiating and heating is preceded by a step of adding a substance to better control the heating and to achieve better welding results, the substance selected from the group consisting of a biological glue, a light-sensitive dyed tissue, a cell and a cooling liquid.

According to one feature in the method of the present invention, the step of irradiating and heating includes irradiating and heating with lasers.

According to another feature in the method of the present invention, the step of controlling the light sources is done by a controller coupled to both the IR radiometer and the light sources and operative to provide the control of each light source based on the radiation inputs.

According to yet another feature in the method of the present invention, the irradiation by the light sources and the thermal radiation from the tissue sample is conducted through a conduit selected from the group consisting of a single port and a plurality of separate ports.

According to yet another feature in the method of the present invention, the single port includes an optical fiber.

According to yet another feature in the method of the present invention, the separate ports include separate optical fibers.

According to yet another feature in the method of the present invention, the lasers are selected from the group consisting of a pulsed laser, a continuous wave gas laser, a solid-state laser, a semiconductor laser and a combination thereof.

According to yet another feature in the method of the present invention, the IR radiometer includes an IR detector selected from the group consisting of a thermal detector and a photonic detector.

BRIEF DESCRIPTION OF THE DRAWINGS

For a better understanding of the present invention and to show more clearly how it could be applied, reference will now be made, by way of example only, to the accompanying drawings in which:

FIG. 1 (A-B) is a schematic diagram of a laser heating and welding temperature controlled system of the present invention in two configurations of conveying the radiation to and from the tissue sample: (A) one port system, and (B) two port system;

FIG. 2 shows schematically the advantage of using a two wavelength laser welding system over a one wavelength laser welding system;

FIG. 3 shows a schematic example of multi-wavelength laser welding temperature controlled fiber optic system utilizing $CO_2$ gas laser and a GaAs diode laser;

FIG. 4 shows a schematic of the synchronization between the lasers irradiation and the thermal measurement;

FIG. 5 shows the temperature control result of a biological tissue irradiated by multi-wavelength laser welding temperature controlled fiber optic system utilizing $CO_2$ gas laser and a GaAs diode laser.

DETAILED DESCRIPTION OF THE PREFERRED EMBODIMENTS

The present invention discloses, in various embodiments, a novel temperature controlled multi-wavelength laser heating and welding system, in which a plurality of different wavelength light sources simultaneously irradiate the tissue sample and in which radiometry is used to measure the temperature of the tissue sample to provide closed loop control of the heating. The radiometric measurements may be performed in several spectral bands. In one embodiment, the system is based on all-fiber technology and on diode lasers. This enables the system to be reliable, compact, rigid and portable, allowing the system to be commercialized for many medical procedures.

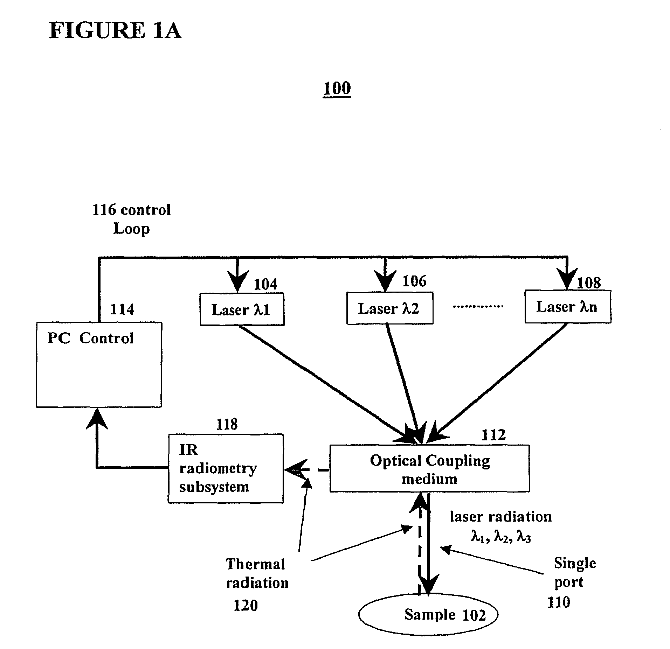

FIG 1A shows schematically a first embodiment of a laser welding system **100** of the present invention. System **100** comprises a plurality of laser sources (in this case, three sources numbered **104, 106** and **108**) disposed to irradiate tissue sample **102** simultaneously at different wavelengths $\lambda_1, \lambda_2, \lambda_3$. Laser sources **104, 106** and **108** may be pulsed lasers, continuous wave gas lasers, solid-state lasers, semiconductor lasers and a combination thereof. In this embodiment, the irradiation occurs through a single port **110** and an optical coupling medium **112**. Each of the laser sources **104**, **106** and **108** is preferably controlled by a personal computer (PC) **114** through a control loop **116**. Other types of controllers capable of control and analysis functions may also be used. Preferably system **100** further comprises a radiometry subsystem **118** disposed to monitor and measure thermal radiation **120** emitted from heated tissue sample **102**. Preferably, in this embodiment, radiation **120** is conveyed to subsystem **118** through the same single port **110** and optical coupling medium **112** as the laser radiation. In other words, in this embodiment single port **110** conveys simultaneously two types of radiation: laser radiation of the laser sources and thermal radiation from the heated tissue sample. In one embodiment, optical coupling medium **112** is a wave-guide, i.e., an optical fiber. In other embodiments, optical coupling medium **112** may be a refractive element, such as lens or prism, or a reflective element such as a mirror.

Subsystem **118** preferably includes an IR detector in optical communication along an optical path with optical coupling medium **112**. Subsystem **118** can measure the IR thermal radiation in one wavelength, two wavelengths or several wavelengths by using filters, a grating, an acousto-optical filter or any other optical element for filtering wavelength [see e.g., S. Sade and A. Katzir, " Spectral Emissivity and Temperature Measurements of Selective Bodies using Multi-band Fiber Optic Radiometry", Journal of Applied Physics, 96, 3507 (2004)]. Subsystem **118** converts the IR radiation into an electrical, signal which is read by PC **114**, which in turn uses it as an input to control lasers sources **104, 106** and **108**.

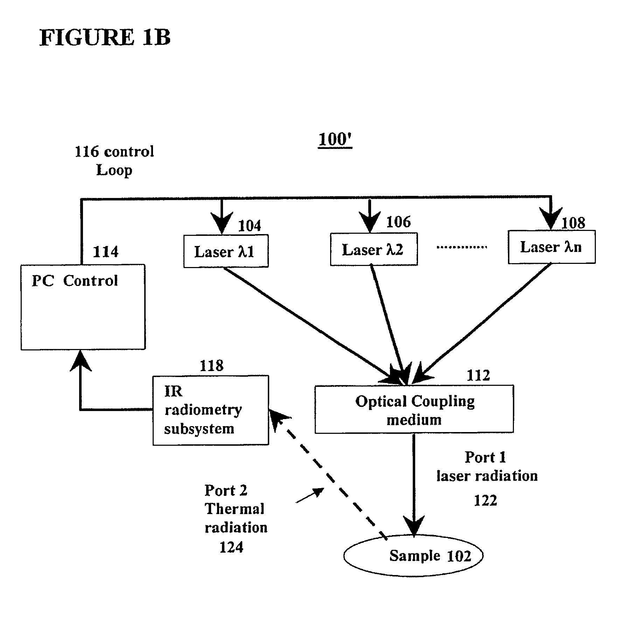

FIG.1B shows schematically a second embodiment of a laser welding system **100'** of the present invention. In contrast with system **100**, system **100'** uses two separate ports **122** and **124**, one for conveying radiation to and the other for receiving radiation from tissue sample **102**. Port **122** is used to convey the multiple laser radiation to tissue sample **102** and port **124** is used to convey the thermal radiation emitted from tissue sample **102** to IR detector subsystem **118**. It is to be understood that in this embodiment, each port is used to convey a different type of radiation independently of the other port. Except for the separate ports, system **100'** includes the same elements as system **100**.

The advantage of such systems **100** and **100'** is as follows. By using several lasers simultaneously, a user can achieve novel and better control over the heating of tissue sample 102. Such improved control is essential for welding and/or heating biological tissues. It is well known that for such tissue samples the absorption length, and therefore the heated region, is wavelength dependent and changes according to the tissue type and structure. In addition, the beams of the different wavelengths can be shaped and /or manipulated in different ways, for example, be focused to a different spot size.

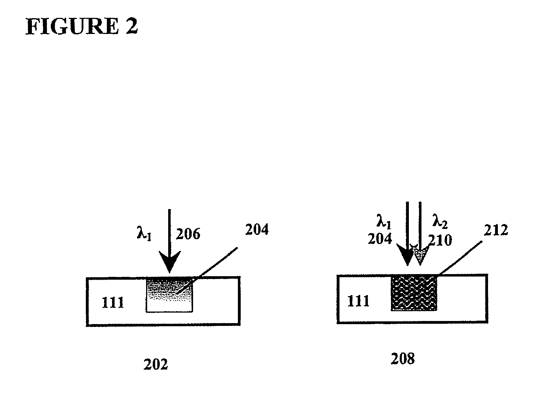

FIG. 2 describes schematically the advantage of using several wavelengths lasers for welding over a single wavelength laser welding system. In the case of laser welding systems which use only one wavelength **202**, heated region **204** which is created by laser $\lambda_1$ **206** is not uniform, i.e. a temperature gradient exist between the upper surface and the inner bulk of the tissue sample **102.** In the case of laser welding systems which uses more than one wavelength **208** (in this example two wavelengths) and assuming that the penetration depth of laser $\lambda_1$ **204** is shorter than the penetration length of laser $\lambda_2$ **210**, laser $\lambda_1$ **204** heats the upper surface and laser $\lambda 2$ **210** heats the inner bulk of the tissue sample **102**. By simultaneously irradiating the tissue sample with two lasers **204** and **210** we obtain simultaneous heating of the upper surface of tissue sample **102** and the inner bulk of tissue sample **102**. We thus achieve a better control of the heated region **212** and parameters such as the heated volume and the uniformity of the heated region **212** can be controlled in a better manner. It is to be understood that this example of using two lasers at two wavelengths is for the purpose of explanation only and that the principle described is the same for using several lasers each radiating at different wavelength.

**Temperature measurement using the IR radiometry sub system 118**

As mentioned, the temperature measurement of the tissue sample **102** is done by subsystem **118** i.e., by radiometric methods. Radiometric methods are based on collecting the infrared radiation emitted from a warm body in a certain spectral band $\Delta\lambda$ and determining the temperature $T$ of the body, using the formula $I = \int_{\Delta\lambda} \varepsilon(\lambda) W_{bb\lambda}(\lambda, T) d\lambda$, where $I$ is the radiance, $\varepsilon$ is the body emissivity, and $W_{bb\lambda}$ is the Planck distribution function [see e.g., M Bass, Handbook of optics, McGraw-Hill, Inc. New-York, 1995, 2nd ed. Chapter 24]. IR radiometers basically consist of an optical part, which collects and delivers the emitted IR radiation from the measured body to an infrared detector. The detector converts the radiation to an electrical signal, which is then, amplified by an electronic circuits. An infrared transmitting fiber can be incorporated in the radiometric system, to collect and deliver the emitted radiation from the measured body to the detector.

The temperature measurement can be done by any of three methods according to the number of spectral bands in which the IR radiation is measured: (I) Single band method - the radiometer measures the radiation in a single spectral band. The assumption is that the emissivity of the measured body is close to 1 (i.e. blackbody). (II) Dual band method - these radiometers measure the radiation emitted in two different spectral bands. It is assumed that the emissivity is constant (i.e. gray body) in both spectral bands and that the background radiation is low relative to the temperature of the tissue sample. The temperature is calculated from the ratio of the signals obtained for the two bands. (III) Multi-band method [see e.g. S. Sade and A. Katzir, " Spectral Emissivity and Temperature Measurements of Selective Bodies using Multi-band Fiber Optic Radiometry", Journal of Applied Physics, 96, 3507 (2004)] - these radiometers measure the IR radiation in at least three spectral bands.

The multi-band method is based on measuring the emitted radiation from tissue sample 102 at different spectral bands in the mid and far infrared 2-30$\mu$m using multi-band radiometer. Then by solving a set of equations, one can measure simultaneously and in real time the tissue sample temperature and emissivity and the background temperature. For N spectral bands we obtain a set of N equations with M=N+2 unknowns: N unknowns are the N emissivities in the N spectral bands, and two unknowns are the two temperatures $T_{sample}$ and $T_{back}$. The set of equations can be written in a general form:

$$S(\Delta\lambda_1) = A_1 \times f(T_{sample}, \varepsilon_1^{sample}, T_{back}) + B_1(\Delta\lambda_1)$$
$$S(\Delta\lambda_2) = A_2 \times f(T_{sample}, \varepsilon_2^{sample}, T_{back}) + B_2(\Delta\lambda_2)$$
$$.$$
$$.$$
$$S(\Delta\lambda_N) = A_N \times f(T_{sample}, \varepsilon_N^{sample}, T_{back}) + B_N(\Delta\lambda_N)$$

$$(1)$$

Experimentally $A_i$ and $B_i$ can be found via calibration measurements using a blackbody (or a body with a known emissivity). The value of the tissue sample emissivity, temperature and the background temperature is obtained simultaneously from the solution of equation set (1).

**APPLICATION EXAMPLE : A temperature controlled multi wavelength laser welding and heating system utilizing $CO_2$ gas laser and a GaAs diode laser.**

In this example we describe experiments and their results. The experiments were done using an experimental two port temperature controlled multi wavelength laser welding and heating system, such as system **100'**.

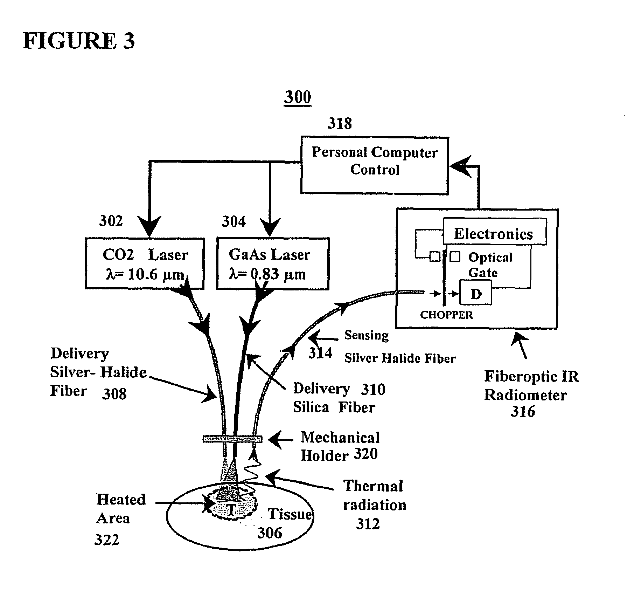

FIG. 3 illustrates an experimental system **300** that uses a $CO_2$ gas laser source **302** and a GaAs diode laser source **304**. The two laser sources were used to irradiate simultaneously a biological tissue sample **306.** $CO_2$ laser **302** emitted radiation at a wavelength of 10.6 micron, and was coupled to a silver halide infrared transmitting optical fiber **308**. The GaAs laser diode **304** emitted radiation at a wavelength of 0.83 micron, and was coupled to a silica fiber **310**. The thermal radiation **312** emitted from the heated tissue sample **306** was collected by a silver halide optical fiber **314** and delivered to an IR radiometer **316**. The signal from radiometer **316** is read by a PC **318**, which uses it to control the power of the two lasers sources.

The Optical Penetration Depth, O.P.D., in water is approximately 11 microns for the $CO_2$ laser and approximately 1 meter for the GaAs diode laser. The O.P.D. of the GaAs laser can be controlled by using indocyanine green at different concentrations. Therefore by simultaneously using both lasers and an adequate concentration of the indocyanine green a versatile light source was formed.

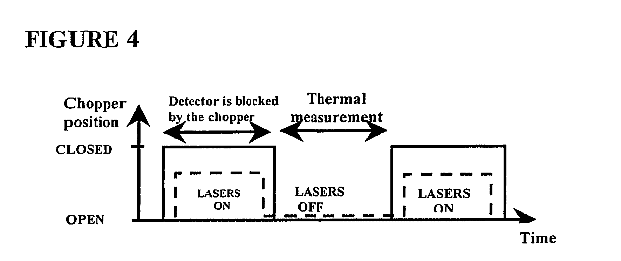

Radiometer 316 was calibrated and used to measure the surface temperature of the tissue sample 306, and a proportional control algorithm [see e.g., O.Eyal, A.Katzir, " Thermal feedback control technique for transistor - transistor logic triggered CO2 laser used for irradiation of biological tissue utilizing infrared fiber optic radiometry," Applied Optics, Vol.33, No.9, 1994] used in the personal computer **318** were used to control the lasers. Radiometer **316** is based on a pyroelectric detector and has an internal chopper. The chopper and a synchronized electric circuit were used to solve the problem of blinding of the detector by the $CO_2$ laser light reflected from the tissue sample. The tissue sample is irradiated by a train of laser pulses synchronized to the time intervals when the detector is blocked by the chopper, whose position is detected by an optical gate. This synchronization, shown in FIG.4, ensures that only thermal radiation can reach the detector, whereas the $CO_2$ laser radiation reflected from the sample is blocked and cannot interfere with the measured signal. The chopper frequency was adjusted to 8 Hz, and therefore this was also the irradiation frequency of the tissue. The average powers of the $CO_2$ and GaAs lasers were adjusted to 0.31 Watt and 1.3 Watt, respectively.

All three optical fibers were held by a mechanical holder **320** 3 mm above the tissue sample and were directed to the same point. The diameter of the irradiated spot **322** was approximately 3 mm, for both laser wavelengths. The length of the silver halide fibers **308** and **314** was 1.5 meter, and it had diameter of 0.9 mm, while the length of the silica fiber **310** was 2 meter and it had diameter of 0.6 mm.

**Experimental results**

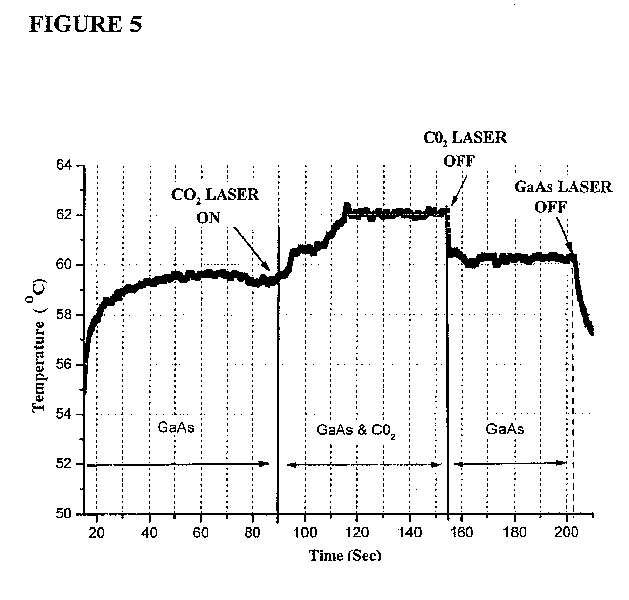

The experimental results are described in FIG. 5. The tissue sample **306** was dyed with indocyanine green at concentration of approximately 1 mg/ml. It was first irradiated just by the GaAs laser **304** and its temperature was elevated from a room temperature of 30°C and stabilized to a target temperature of 60 $\pm$ 1 °C. While GaAs laser **304** was still working, $CO_2$ laser **302** was set to ON and the tissue temperature was stabilized to a target temperature of 62 $\pm$ 1°C. The $CO_2$ radiation was then stopped and the temperature was stabilized back to the target temperature of 60 $\pm$ 1 °C. Finally, GaAs laser **304** was stopped and tissue **306** cooled down to its initial room temperature. The temperature profile of the tissue is affected by changes that occur in the tissue structure during the irradiation procedure, and can be better controlled by choosing the right form and parameters for the control algorithm.

In summary, the present invention discloses a novel system and method for welding, soldering and heating of samples, in particular tissue samples, in which the temperature of the tissue is being measured and monitored using IR radiometry for providing respective inputs for a closed loop control of a plurality of light sources, which in turn irradiate simultaneously the tissue sample. Each light source has a different wavelength, which means that the tissue sample is being irradiated simultaneously by radiation of several wavelengths, giving rise to better control of the heated region and volume making the welding, soldering and heating processes more efficient. Moreover, the temperature measurement can be done in several wavelengths, which makes the temperature measurement more accurate.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

While the invention has been described with respect to a limited number of embodiments, it will be appreciated that many variations, modifications and other applications of the invention may be made.

**Claims**

1.    A system for non-contact welding, soldering and heating of a tissue sample, comprising:

   a. a plurality of light sources each having a different wavelength, the sources operative to simultaneously irradiate the tissue sample thereby causing heating; and
   b. an IR radiometry subsystem for monitoring the IR radiation emitted from the tissue sample during the heating and for providing respective IR radiation inputs for control of each of the light sources.

2.    The system of claim 1, wherein the light sources include lasers.

3.    The system of claim 2, further comprising a control unit coupled to both the IR radiometry subsystem and the laser sources and operative to provide the control of each laser source based on the radiation inputs.

4.    The system of claim 1, wherein the irradiation by the light sources and the thermal radiation from the tissue sample are conducted through a single port.

5. The system of claim 1, wherein the irradiation by the light sources and the thermal radiation from the tissue sample are conducted through separate ports.

6. The system of claim 4, wherein the single port includes an optical fiber.

7. The system of claim 5, wherein the separate ports include separate optical fibers.

8. The system of claim 6, wherein the lasers are selected from the group consisting of a pulsed laser, a continuous wave gas laser, a solid-state laser, a semiconductor laser and a combination thereof.

9. The system of claim 1, wherein the IR radiometry subsystem includes an IR detector selected from the group consisting of a thermal detector and a photonic detector.

10. The system of claim 9, wherein the IR radiometry subsystem further includes an IR-transparent optical fiber for coupling the IR detector to the tissue sample along an optical path.

11. A non-contact welding, soldering and heating system comprising:

    a. a plurality of light sources for simultaneously and controllably irradiating and heating a tissue sample;
    b. an infrared detector for monitoring IR radiation emitted from the tissue sample during the heating in at least one spectral band and for providing respective IR radiation inputs; and
    c. a controller for controlling each of the light sources based on the IR radiation inputs, thereby facilitating the controllable heating of the tissue sample.

12. A method for controllably welding, soldering and heating of a tissue sample comprising the steps of:

    a. irradiating and heating the tissue sample simultaneously using a plurality of light sources;
    b. measuring the temperature of the tissue sample using an IR radiometer in at least one spectral band to provided real-time temperature inputs; and
    c. controlling each of the light sources based on real-time temperature inputs provided by the IR radiometer; thereby providing a controlled tissue sample temperature.

13. The method of claim 12, wherein the step of irradiating and heating is preceded by a step of adding a substance to better control the heating and to achieve better welding results, the substance selected from the group consisting of a biological glue, a light-sensitive dyed tissue, a cell and a cooling liquid.

14. The method of claim 12, wherein the step of irradiating and heating includes irradiating and heating with lasers.

15. The method of claim 12, wherein the step of controlling the light sources is done by a controller coupled to both the IR radiometer and the light sources and operative to provide the control of each light source based on the radiation inputs.

16. The method of claim 12, wherein the irradiation by the light sources and the thermal radiation from the tissue sample is conducted through a conduit selected from the group consisting of a single port and a plurality of separate ports.

17. The method of claim 16, wherein the single port includes an optical fiber.

18. The method of claim 16, wherein the separate ports include separate optical fibers.

19. The method of claim 17, wherein the lasers are selected from the group consisting of a pulsed laser, a continuous wave gas laser, a solid-state laser, a semiconductor laser and a combination thereof.

20. The method of claim 12, wherein the IR radiometer includes an IR detector selected from the group consisting of a thermal detector and a photonic detector.

# FIGURE 1A

<u>100</u>

**116 control
Loop**

**114**

**PC Control**

**104** Laser λ1

**106** Laser λ2

............

**108** Laser λn

**118**
**IR
radiometry
subsystem**

**112**
**Optical Coupling
medium**

**laser radiation**
$\lambda_1, \lambda_2, \lambda_3$

**Thermal
radiation
120**

**Single
port
110**

**Sample 102**

# FIGURE 1B

**100'**

116 control
Loop

114

PC Control

104
Laser λ1

106
Laser λ2 ·············

108
Laser λn

118
IR
radiometry
subsystem

112
Optical Coupling
medium

Port 1
laser radiation

122

Port 2
Thermal
radiation
124

Sample 102

# FIGURE 2

$\lambda_1$ | 206    204

111

202

$\lambda_1$   $\lambda_2$
204   210   212

111

208

**FIGURE 3**

# FIGURE 4

**FIGURE 5**

**European Patent Office**

## PARTIAL EUROPEAN SEARCH REPORT

which under Rule 45 of the European Patent Convention shall be considered, for the purposes of subsequent proceedings, as the European search report

Application Number

EP 07 00 3129

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/78830 A (MEDELASER LLC [US]; CASEY SEAN M [US]; GERDES HAROLD M [US]) 25 October 2001 (2001-10-25) * page 12, line 17 - page 15, line 11; figure 1 * | 1-3,5,9,11 | INV. A61B18/22 ADD. A61B17/00 |
| X | US 5 662 643 A (KUNG ROBERT T V [US] ET AL) 2 September 1997 (1997-09-02) * column 2, line 46 - column 3, line 7 * * column 5, line 58 - line 67; figures 1-3 * | 1-11 | |
| Y | US 5 334 191 A (POPPAS DIX P [US] ET AL) 2 August 1994 (1994-08-02) * column 7, line 26 - column 8, line 8; figure 4a * | 1-11 | |
| Y | US 6 162 213 A (STEWART BOB W [US]) 19 December 2000 (2000-12-19) * column 15, line 28 - line 59 * | 1-11 | |
| A | EP 0 368 512 A (PFIZER HOSPITAL PROD [US]) 16 May 1990 (1990-05-16) * column 8, line 32 - line 43 * | 1,11 | TECHNICAL FIELDS SEARCHED (IPC) A61B |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 27 July 2007 | MAYER-MARTENSON, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&: member of the same patent family, corresponding document

EPO FORM 1503 03.82 (P04C07)

**European Patent Office**

**INCOMPLETE SEARCH**

**SHEET C**

Application Number

EP 07 00 3129

Claim(s) searched completely:
        1-11

Claim(s) not searched:
        12-20

Reason for the limitation of the search (non-patentable invention(s)):

Article 52 (4) EPC - Method for treatment of the human or animal body by surgery

## ANNEX TO THE EUROPEAN SEARCH REPORT
## ON EUROPEAN PATENT APPLICATION NO.

EP 07 00 3129

This annex lists the patent family members relating to the patent documents cited in the above-mentioned European search report.
The members are as contained in the European Patent Office EDP file on
The European Patent Office is in no way liable for these particulars which are merely given for the purpose of information.

27-07-2007

| Patent document cited in search report | | Publication date | Patent family member(s) | | Publication date |
|---|---|---|---|---|---|
| WO 0178830 | A | 25-10-2001 | AU | 5706901 A | 30-10-2001 |
| US 5662643 | A | 02-09-1997 | NONE | | |
| US 5334191 | A | 02-08-1994 | US | 5409481 A | 25-04-1995 |
| US 6162213 | A | 19-12-2000 | NONE | | |
| EP 0368512 | A | 16-05-1990 | AU | 626339 B2 | 30-07-1992 |
| | | | AU | 4452089 A | 02-08-1990 |
| | | | CA | 2002453 A1 | 10-05-1990 |
| | | | DK | 560389 A | 11-05-1990 |
| | | | JP | 2177954 A | 11-07-1990 |
| | | | NO | 894469 A | 11-05-1990 |
| | | | PT | 92240 A | 31-05-1990 |
| | | | US | 5540676 A | 30-07-1996 |
| | | | US | 5139494 A | 18-08-1992 |
| | | | US | 5304167 A | 19-04-1994 |
| | | | ZA | 8908555 A | 30-10-1991 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5409481 A
- US 20030055413 A
- US 6162213 A
- US 6607522 B
- US 5552452 A
- US 4633870 A

### Non-patent literature cited in the description

- Laser Tissue Welding. **KAREN M.MCNALLY-HEINTZELMAN ; ASHLEY J.WELCH.** Biomedical Photonics Handbook. Tuan Vo-Dinh, 2003
- **L.S.BASS ; M.R. TREAT.** Laser Tissue Welding: A Comprehensive Review of Current and Future Clinical Applications. *Lasers in Surgery and Medicine,* 1995, vol. 17, 315-349
- **S. SADE ; O. EYAL ; V. SCHARF ; A. KATZIR.** Fiberoptic Infrared Radiometer for Accurate Temperature Measurements. *Applied Optics,* 2002, vol. 41 (10), 1908-1914
- **A ZUR ; A. KATZIR.** Use of infrared fibers for low temperature radiometric measurements. *Applied Physics Letters,* 1986, vol. 48, 449
- **S. SADE ; A. KATZIR.** Spectral Emissivity and Temperature Measurements of Selective Bodies using Multi-band Fiber Optic Radiometry. *Journal of Applied Physics,* 2004, vol. 96, 3507
- **M BASS.** Handbook of optics. McGraw-Hill, Inc, 1995
- **O.EYAL ; A.KATZIR.** Thermal feedback control technique for transistor - transistor logic triggered CO2 laser used for irradiation of biological tissue utilizing infrared fiber optic radiometry. *Applied Optics,* 1994, vol. 33 (9